# EUROPÄISCHE PATENTSCHRIFT

(11) **EP 2 208 461 B1**
(45) Veröffentlichungstag und Bekanntmachung des Hinweises auf die Patenterteilung: **12.12.2018**
(21) Anmeldenummer: 10000126.2
(22) Anmeldetag: 08.01.2010
(51) Int. Cl.: A61B 5/0408, A61N 1/04

(54) **Verfahren zur Herstellung einer Bioelektrode**
Method of manufacturing a bioelectrode
Procédé de fabrication d'une bioélectrode

(30) Priorität: 20.01.2009 AT 852009; 27.07.2009 AT 11732009
(43) Veröffentlichungstag der Anmeldung: 21.07.2010
(73) Patentinhaber: Leonh. Lang, 6020 Innsbruck (AT)
(72) Erfinder: Lang, Burrhus, 6020 Innsbruck (AT); Wilfinger, Markus, 6063 Rum (AT)
(74) Vertreter: Torggler & Hofinger Patentanwälte

(56) Entgegenhaltungen:
- WO-A2-2008/017921
- US-A- 3 547 105
- US-A- 5 269 810
- US-A1- 2005 015 134
- US-A1- 2006 040 427
- "Typical Properties for 3M Electrically Conductive Adhesive Films" INTERNET CITATION 1. Februar 2000 (2000-02-01), XP002572097 Gefunden im Internet: URL:http://multimedia.3m.com/mws/mediawebs erver?66666UuZjcFSLXTtmXfVMxT2E VuQEcuZgVs6EVs6E666666--> [gefunden am 2010-03-09]

## Beschreibung

Die Erfindung betrifft Verfahren zur Herstellung einer Bioelektrode gemäß Oberbegriff des Anspruchs 1.

Bioelektroden kommen in vielfacher Weise zum Einsatz. Es wird entweder wie bei Defibrillationselektrode und Stimulationselektrode Strom dem menschlichen oder tierischen Körper zugeführt oder Strom vom Körper abgeführt (beispielsweise Neutralelektroden oder Messelektroden).

Gattungsfremde Bioelektroden ohne aufgefächerte Litzen gehen aus US 3,547,105 und der US 5,269,810 hervor. Dagegen zeigen die US 2005/0015134 A1 und die US 2006/0040427 A1 gattungsbildende Bioelektroden mit aufgefächerten Litzen.

Aufgabe der Erfindung ist es, ein Verfahren zur Herstellung einer Bioelektrode zu schaffen, bei dem das elektrische Anschlusskabel einen guten mechanischen Halt in der Elektrode hat und außerdem ein guter elektrischer Kontakt zu jenen Schichten der Elektrode gewährleistet ist, die letztlich den Strom der Haut zuführen oder von dieser abnehmen.

Erfindungsgemäß wird dies durch ein Verfahren gemäß Anspruch 1 erreicht.

Das erfindungsgemäß hergestellte elektrische Leitelement lässt sich vor dem Zusammenbau der eigentlichen Bioelektrode in einem gesonderten Arbeitsprozess mechanisch fest und elektrisch gut leitend mit dem elektrischen Anschlusskabel verbinden. Bevorzugt besteht das elektrische Leitelement aus einer flächigen Schicht aus elektrisch leitfähigem, thermoplastischem Material. Das Ende des Anschlusskabels kann beispielsweise von einer aushärtbaren Masse umgeben sein und so das vormontierte elektrische Leitelement bilden. Es ist aber auch möglich, eine bereits vorhandene thermoplastische Schicht mit dem Ende des Anschlusskabels thermisch zu verschweißen oder mittels Ultraschalleinwirkung zu verschweißen. In jedem Fall hat man eine im Verhältnis zum Leiter des Anschlusskabels vergrößerte elektrisch leitende Fläche des elektrischen Leitelementes. Hiermit lässt sich das vormontierte elektrische Leitelement gut in der Bioelektrode verankern.

Das vormontierte elektrische Anschlusselement erlaubt es auch, den über das elektrische Anschlusskabel zugeführten Strom über eine größere Fläche gleichmäßig zu verteilen bzw. von einer größeren Fläche abzunehmen. Es ist sogar möglich, in der elektrisch leitenden Schicht des Anschlusselementes, die auch aus mehreren Unterschichten bestehen kann, ein spezielles Widerstandsprofil einzubauen, beispielsweise derart, dass der Flächenwiderstand vom mittleren Anschlusspunkt des elektrodenseitigen Kabelendes zum Rand hin je nach Bedarf abnimmt oder zunimmt. Jedenfalls ist eine gezielte flächige Stromverteilung möglich. Weitere Vorteile und Einzelheiten der Erfindung werden anhand der nachfolgenden Figurenbeschreibung näher erläutert.

Die Fig. 1a zeigt ein erstes erfindungsgemäß hergestelltes Ausführungsbeispiel in einer schematischen Explosionsdarstellung. Fig. 1b zeigt die Elektrode der Fig. 1a in einer schematischen Querschnittsdarstellung. Fig. 1c zeigt einen Kabelquerschnitt.

Die Fig. 2a zeigt ein zweites erfindungsgemäß hergestelltes Ausführungsbeispiel in einer schematischen Explosionsdarstellung. Fig. 2b zeigt die Elektrode der Fig. 2a in einer schematischen Querschnittsdarstellung.

Die Fig. 3a zeigt ein drittes erfindungsgemäß hergestelltes Ausführungsbeispiel in einer schematischen Explosionsdarstellung. Fig. 3b zeigt die Elektrode der Fig. 3a in einer schematischen Querschnittsdarstellung.

Die Fig. 4a zeigt ein viertes erfindungsgemäß hergestelltes Ausführungsbeispiel in einer schematischen Explosionsdarstellung. Fig. 4b zeigt die Elektrode der Fig. 4a in einer schematischen Querschnittsdarstellung.

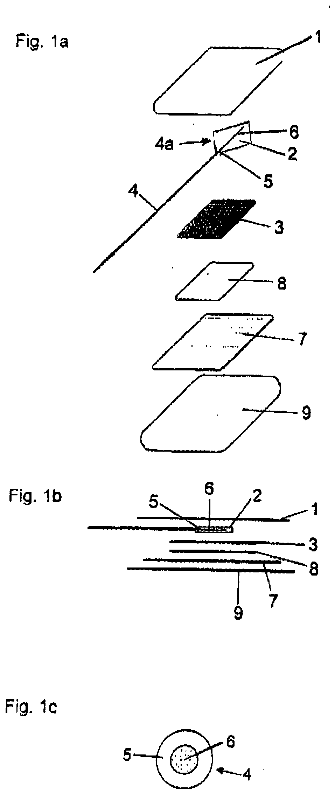

Die Fig. 1a und 1b zeigen ein erstes Ausführungsbeispiel einer erfindungsgemäß hergestellten Elektrode, insbesondere Defibrillationselektrode.

Die Elektrode der Fig. 1a und 1b weist unterhalb eines Trägermateriales 1 (z.B.: Schaummaterial bestehend aus Polyethylen oder ähnlichem, Folie bestehend aus Polyethylenterephthalat oder ähnlichem) eine thermoplastische Schicht 3 auf, die elektrisch leitend ausgebildet ist. Die thermoplastische Schicht (3) kann z.B.: aus Polyvinylchlorid, Acrylbutadienstyrol, Polyurethan, Polyethylen oder ähnlichem bestehen. Die elektrische Leitfähigkeit der thermoplastischen Schicht 3 kann beispielsweise durch metallische Einschlüsse und/oder Einschlüsse auf Kohlenstoffbasis (Ruß, Graphit) erzielt werden. Durch eine entsprechende Verteilung dieser Einschlüsse oder eine Mehrschichtigkeit der Schicht 3 kann man den elektrischen Widerstand über die Elektrode variieren.

Erfindungsgemäß ist am elektrodenseitigen freien Ende 4a des Anschlusskabels 4 ein elektrisches Leitelement vormontiert. Bei dem in Fig. 1a und 1b gezeigten Ausführungsbeispiel besteht das elektrische Leitelement 2 aus einer ausgehärteten (vorzugsweise thermoplastischen) elektrisch leitenden Massen, die mechanisch fest und elektrisch in gut leitendem Kontakt mit dem Kabelende 4a verbunden ist. Die elektrisch leitende Masse, in der das Kabelende angeordnet, vorzugsweise eingegossen ist, ist erfindungsgemäß schichtförmig ausgebildet. Diese flächige Ausbildung erlaubt einen guten Einbau in die Bioelektrode, wobei bei einem guten mechanischen Halt auch ein guter elektrischer Kontakt (in Fig. 1a zur elektrisch leitenden Schicht 3) herstellbar ist. Dabei weist das elektrische Leitelement 2 eine geringe Bauhöhe auf. Geringe Bauhöhe heißt in diesem Fall, dass erfindungsgemäß das elektrische Leitelement 2 gemeinsam mit dem Kabelende 4a eine Schichtdicke zwischen 50 und 250 Mikrometer, vorzugsweise zwischen 50 und 200 Mikrometer, aufweist. Diese gemeinschaftliche Schichtdicke vom Kabelende 4a und Leitelement 2 gilt in der gesamten Bioelektrode, das heißt im Bereich zwischen der hautseitigen Klebeschicht 7 und einem nicht leitendem Trägermaterial 1. Wenn die Schichtdicke unter 50 Mikrometer liegen würde, würde dies zu Problemen beim Verschweißen führen. Bei zu dickem Leitelement (über 250 Mikrometer) ist die Flexibilität der Elektrode nicht mehr gewährleistet, das heißt die Anpassungsfähigkeit an den menschlichen Körper geht verloren bzw. wird vermindert.

Es wird darauf hingewiesen, dass die Querschnittsdarstellung gemäß der Fig. 1b nur die Schichtfolge anzeigt, die einzelnen Schichten der Elektrode aber natürlich direkt aneinander liegen und miteinander verbunden sind.

Das elektrische Anschlusskabel 4 umfasst - wie in Fig. 1c im Querschnitt dargestellt - einen elektrisch isolierenden Kabelmantel 5, in dem sich zumindest ein elektrischer Leiter 6 befindet. Dieser elektrische Leiter 6 ist eine Litze, die aus mehreren Einzeldrähten/Einzelfasern besteht.

Verwendbare Materialien des isolierenden Kabelmantels sind Polyethylen, Polypropylen oder Polyvinylchlordid oder ähnliches. Die Leitadern können Karbonfaserstränge sein, die aus mehreren 1000 bis mehreren 10.000 Einzelfasern bestehen, welche metallisiert sein können. Als Leitader können auch Metalllitzen alleine oder Metalllitzen kombiniert mit Karbonfasern verwendet werden.

Eine derartige Kabelausbildung erlaubt es, das Kabelende beispielsweise auf einer Länge von 0,5 cm bis 5 cm abzuisolieren, sodass der Leiter 6 freiliegt. Das vormontierte elektrische Leitelement 2 wird dann vorzugsweise nicht nur mit dem abisolierten Leiterbereich, sondern auch mit einem Teil des isolierenden Kabelmantels 5 verbunden. Hiermit kann man den mechanischen Halt zwischen dem Kabelende 4a und dem elektrischen Leitelement noch weiter erhöhen. Erfindungsgemäß ist vorgesehen, die Einzeldrähte der Litze des Leiters aufzufächern und somit einen verbesserten Halt und einen verbesserten elektrischen Kontakt in der Vergussmassenschicht des elektrischen Leitelements 2 zu erzielen.

Im Übrigen sei erwähnt, dass die elektrische Leitfähigkeit der vorzugsweise thermoplastischen Schicht des elektrischen Leitelements 2 wie schon bei der Schicht 3 durch metallische Einschlüsse und/oder Einschlüsse auf Kohlenstoffbasis (Ruß, Graphit) erzielt werden kann. Durch einen entsprechende Verteilung dieser Einschlüsse oder eine Mehrschichtigkeit des elektrischen Leitelements 2 kann man auch den elektrischen Widerstand über einen gewissen Flächenbereich variieren.

Die Schichtstärke des elektrischen Leitelementes liegt vorzugsweise in der Größenordnung von 100 bis 250 Mikrometer. Diese Schichtstärke gilt sowohl für das elektrische Leitelement 2 alleine als auch für das elektrische Leitelement 2 samt darin oder daran angeordnetem Kabel 4 bzw. elektrischem Leiter 6. Wie in Fig. 1b, 2b und 3b dargestellt wird das Kabelende 4a bzw. der elektrische Leiter 6 vom elektrischen Leitelement 2 umschlossen. Es soll jedoch nicht ausgeschlossen sein, dass dieser elektrische Leiter 6 bzw. das Kabelende 4a auf bzw. unter dem elektrischen Leitelement 2 an diesem anliegt, wobei die Gesamtschichtdicke dennoch 250 Mikrometer nicht übersteigt. Dies bringt vor allem gegenüber der aus dem Stand der Technik bekannten EP 0 337 667 B1 den Vorteil der weit größeren Flexibilität und der viel geringeren Maximaldicke der Bioelektrode.

Hautseitig weist die Bioelektrode nach den Fig. 1a bis 1b eine leitfähige Klebeschicht 7, vorzugsweise in Form eines leitfähigen Gels, auf. Die leitfähige Klebeschicht, welche biokompatibel sein muss, kann sowohl ein klebendes Hydrogel, als auch ein leitfähiger Kleber sein.

Zwischen der hautseitigen leitfähigen Klebeschicht 7 und der elektrisch leitenden thermoplastischen Schicht 3 kann eine Metallschicht oder eine Metall-/Metallchloridschicht angeordnet sein, wobei das Metall vorzugsweise Silber ist. Diese Schicht trägt das Bezugszeichen 8.

Unterhalb der leitfähigen Klebeschicht 7 ist ein abziehbares Abdeckmaterial 9 angeordnet, das die leitfähige Klebeschicht beim Transport und der Lagerung schützt, und das vor Benutzung abgezogen wird. Dieses Abdeckmaterial kann aus Kunststoffen wie Polyethylenterephthalat, Polystyrol, Polypropylen oder ähnlichem bestehen, welche auch silikonisiert sein können.

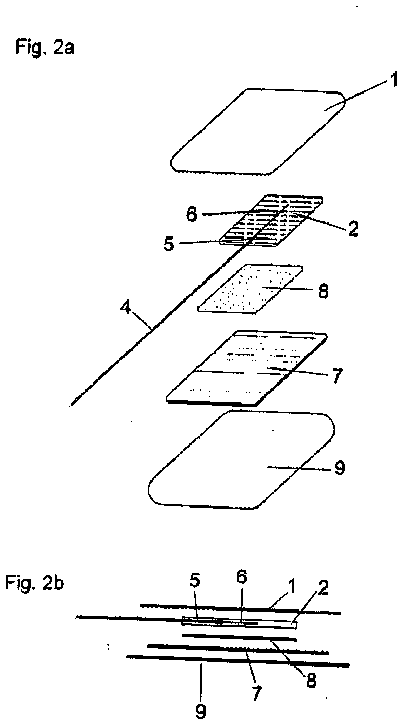

Das zweite Ausführungsbeispiel gemäß den Fig. 2a und 2b unterscheidet sich vom ersten Ausführungsbeispiel gemäß den Fig. 1a und 1b im Wesentlichen durch eine andere Ausbildung des vormontierten elektrischen Leitelementes 2 und durch die Tatsache, dass die elektrisch leitende thermoplastische Zwischenschicht 3 fehlt, sodass das vormontierte elektrische Leitelement direkt auf der Schicht 8 aufliegt. Bei diesem Ausführungsbeispiel gemäß den Fig. 2a und 2b weist das vormontierte elektrische Leitelement eine verhältnismäßig große Fläche auf, die bereits mehr als 50 %, vorzugsweise mehr als 70 %, der Fläche der hautseitigen elektrisch leitenden Klebeschicht 7 beträgt.

Außerdem ist bei dem in den Fig. 2a und 2b dargestellten Ausführungsbeispiel das elektrische Leitelement 2 einem dem Elektrodenbau vorgeschalteten Schritt dadurch hergestellt worden ist, dass der elektrische Leiter des Anschlusskabels 4 mit einer thermoplastischen, elektrisch leitfähigen Schicht verschweißt worden ist. Zum Verschweißen sind erfindungsgemäß thermische Verschweißprozesse oder Ultraschall-Verschweißprozesse. In jedem Fall wird sichergestellt, dass das Kabelende eine gute mechanische Verbindung mit der elektrisch leitfähigen Schicht des vormontierten elektrischen Leitelements 2 aufweist.

Die elektrischen Leitelemente gemäß der Erfindung lassen sich in einem gesonderten Arbeitsprozess in großer Stückzahl verfahrensmäßig optimiert herstellen. Bei der anschließenden eigentlichen Herstellung der Elektrode, nämlich dem Zusammenfügen der Schichten gemäß den Figuren 1a, 2a bzw. 3a durch verschweißen oder verkleben, können diese vormontierten elektrischen Leitelemente samt Kabelende dann einfach eingesetzt werden, was eine rasche Herstellung mit hoher Taktfrequenz erlaubt.

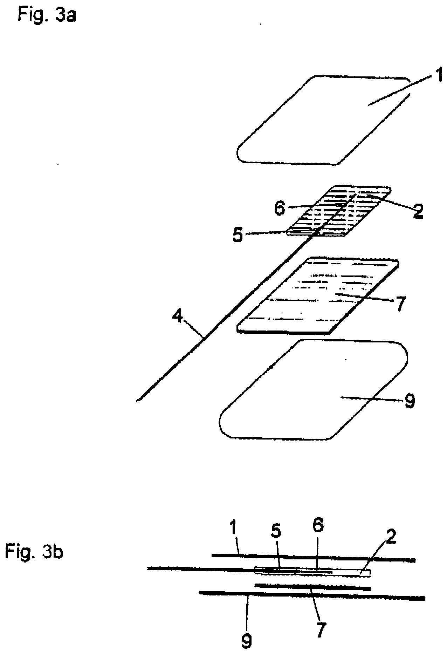

Beim dritten Ausführungsbeispiel gemäß den Fig. 3a und 3b fehlt gegenüber dem zweiten Ausführungsbeispiel gemäß den Fig. 2a und 2b die Schicht 8. Damit liegt das vormontierte elektrische Leitelement 2 direkt auf der Oberseite der hautseitigen elektrisch leitenden Klebeschicht 7 auf. Das Ausführungsbeispiel gemäß den Fig. 3a und 3b zeichnet sich durch eine kleine Schichtanzahl auf. Der Aufbau ist besonders einfach und damit kostengünstig. Dennoch wird durch das vormontierte elektrische Leitelement 2 ein guter mechanischer Halt des Kabelendes in der Elektrode gewährleistet. Außerdem kann durch eine entsprechende flächige Gestaltung des vormontierten elektrischen Leitelements 2 eine gute Stromverteilung über die hautseitige elektrisch leitende Klebeschicht 7 erzielt werden.

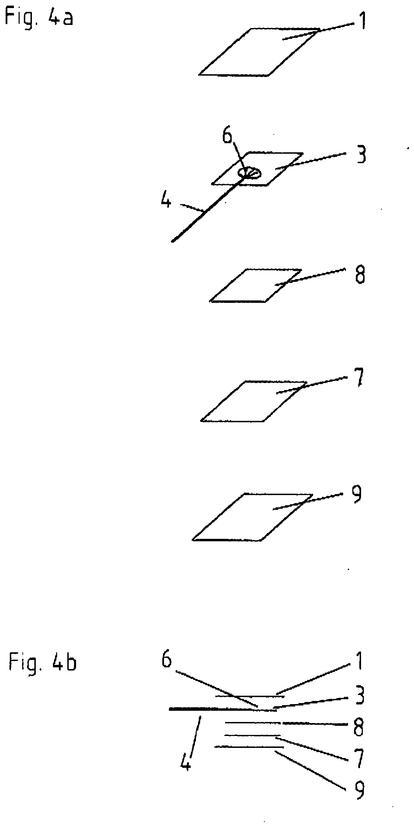

Beim vierten Ausführungsbeispiel gemäß den Fig. 4a und 4b wird das vormontierte elektrische Leitelement durch die Schicht 3 gebildet. In Bezug auf dieses Ausführungsbeispiel gibt es zwei unterschiedliche Herstellungsverfahren.

In einer ersten Variante werden die aufgefächerten Einzeldrähte der Litze des Leiters mittels einer elektrisch leitfähigen Schicht aus Karbonlack mit dem vormontierten elektrischen Leitelement 3 verbunden. Der Lack trocknet und hält dadurch die Einzeldrähte der Litze innig am vormontierten elektrischen Leitelement 3. Dieser Lack kann aus Polyurethan- oder Polyvinylchloridbindemittel bestehen oder diese Bestandteile aufweisen. Um die Leitfähigkeit des Lackes selbst zu ermöglichen, kann dieser Füllkörper wie beispielsweise Karbonfasern, Ruß, Graphit, Metallpigmente usw. umfassen.

Alternativ dazu kann man das elektrische Leitelement 3, wenn dieses als bindemittelhaltige Folie (beispielsweise aus Karbonfolie) ausgebildet ist, in jenem Bereich, wo die Einzeldrähte der Litze befestigt werden sollen, durch ein geeignetes Lösungsmittel anlösen, wodurch das Bindemittel der Folie die Aufgabe des oben beschriebenen Lackes übernimmt. Als Lösungsmittel können Esther oder Ketone verwendet werden.

In beiden Fällen wird keine weitere elektrisch leitende Schicht 8 benötigt. Dennoch kann es von Vorteil sein (für die Stromverteilung und das Handling), eine weitere elektrisch leitende und vorzugsweise thermoplastisch verschweißbare Schicht 8 zu verwenden.

Das erfindungsgemäße Verfahren zeichnet sich in einer weiteren Variante dadurch aus, dass zunächst am elektrodenseitigen Kabelende 4a ein vorzugsweise schichtförmiges elektrisches Leitelement 2 angebracht wird. Erst anschließend wird dieses vormontierte elektrische Leitelement mit mindestens einer weiteren Schicht der Bioelektrode verbunden, vorzugsweise durch verschweißen oder verkleben.

Wie eigentlich aus allen Darstellungen ersichtlich, ist das elektrische Leitelement 2 im Wesentlichen mittig in der Bioelektrode angeordnet, das heißt dass das elektrische Leitelement 2 allseitig vom Rand der Bioelektrode bzw. der äußersten Schichten 1 und 7 bzw. 1 und 9 beabstandet ist. Dieser Abstand sollte umseitig möglichst gleichbleibend sein. Bevorzugt beträgt dieser Abstand zwischen 3 und 20 mm, vorzugsweise zwischen 5 und 15 mm. Durch diese Anordnung des elektrischen Leitelements 2 möglichst in der Mitte der gesamten Bioelektrode ergibt sich eine sehr gute Stromverteilung über den gesamten Bioelektrodenbereich bzw. insbesondere auf die elektrisch leitende Klebeschicht 7.

Bevorzugt kann vorgesehen sein, dass das thermoplastische Material des vormontierten elektrischen Leitelements und/oder der Zwischenschicht durch metallische Einschlüsse und/oder Einschlüsse auf Kohlenstoffbasis elektrisch leitend ist. Weiters kann gemäß einer bevorzugten Ausführungsform vorgesehen sein, dass die elektrisch leitende hautseitige Klebeschicht aus einem leitfähigen Hydrogel oder elektrisch leitendem Kleber besteht. Um eine noch bessere Stromeinleitung zu erreichen, kann vorgesehen sein, dass oberhalb der hautseitigen, elektrisch leitenden Klebeschicht eine Metallschicht oder eine Metall-/Metallchloridschicht angeordnet ist, wobei dieses Metall Silber ist.

Weiters kann bevorzugt vorgesehen sein, dass unterhalb der leitfähigen Klebeschicht ein abziehbares Abdeckmaterial angeordnet ist und/oder auf der der Haut abgewandten Oberseite der Elektrode ein elektrisch nicht leitendes Trägermaterial, vorzugsweise aus Kunststoff, angeordnet ist.

Um besonders hilfreich bei Wiederbelebungsversuchen zu sein, kann vorgesehen sein, dass die Bioelektrode eine Defibrillationselektrode ist, wobei die Fläche jener Schicht die auf der der Haut abgewandten Seite der hautseitigen, elektrisch leitenden Klebeschicht angeordnet ist, vorzugsweise mindestens 50 cm² groß ist.

Die Erfindung ist selbstverständlich nicht auf die dargestellten Ausführungsbeispiele beschränkt. Beispielsweise eignet sich die Erfindung nicht nur für Defibrillationselektroden und Elektroden, die Strom der Haut zuführen (z.B.: Stimulationselektroden), sondern grundsätzlich auch für Elektroden, die Strom von der Haut abnehmen (beispielsweise Neutralelektroden, Messelektroden). Auch der Schichtaufbau und die Größenverhältnisse können von den gezeigten Ausführungsbeispielen abweichen. Wesentlich ist, dass am elektrodenseitigen Ende des Anschlusskabels ein elektrisches Leitelement vormontiert wird, über das ein guter mechanischer Halt und eine gute elektrische Verbindung in der Elektrode gewährleistet sind.

## Patentansprüche

1. Verfahren zum Herstellen einer Bioelektrode, mit einer hautseitigen, elektrisch leitenden Klebeschicht (7) und mit einem flexiblen elektrischen Anschlusskabel (4), das in einem elektrisch isolierenden Kabelmantel (5) zumindest einen elektrischen Leiter (6) in Form einer aus mehreren Einzeldrähten bzw. leitenden Einzelfasern bestehenden Litze umfasst, **gekennzeichnet durch** die Schritte:
- Auffächern der Einzeldrähte bzw. Einzelfasern der Litze,
- Vormontieren eines elektrischen Leitelements (2, 3) am elektrodenseitigen Ende (4a) des Anschlusskabels (4), wodurch das Leitelement (2, 3) mit den aufgefächerten Einzeldrähten bzw. Einzelfasern der Litze des elektrischen Leiters (6) des Anschlusskabels (4) verbunden wird, wobei das elektrische Leitelement (2, 3) und der darin angeordnete elektrische Leiter (6) in der Bioelektrode schichtförmig, mit einer gemeinsamen Schichtdicke zwischen 50 und 250 Mikrometer ausgebildet sind, und
- anschließend elektrisches Verbinden des vormontierten elektrischen Leitelements (2, 3) - gegebenenfalls unter Zwischenschaltung mindestens einer weiteren elektrisch leitenden Schicht (3, 8) - mit der hautseitigen, elektrisch leitenden Klebeschicht (7),
wobei zusätzlich durchgeführt wird:
- dass das vormontierte elektrische Leitelement (2, 3) aus einer elektrisch leitfähigen Schicht gebildet wird, dass die Schicht des vormontierten elektrischen Leitelementes (2) aus einem elektrisch leitfähigen thermoplastischen Material besteht und dass der elektrische Leiter (6) des Anschlusskabels (4) mit der thermoplastischen, elektrisch leitfähigen Schicht verschweißt wird, wobei die verschweißte Schicht das vormontierte elektrische Leitelement (2) bildet,
oder
- Versehen des elektrischen Leitelements (3) oder des elektrischen Leiters (6) des Anschlusskabels (4) mit einer elektrisch leitfähigen Lackschicht und Auflegen des elektrischen Leiters (6) des Anschlusskabels (4) auf das elektrische Leitelement (3), sodass der elektrische Leiter (6) durch das Trocknen der Lackschicht mit dem elektrischen Leitelement (3) verbunden wird, wobei der Lack der Lackschicht aus Polyurethanbindemittel oder Polyvinylchloridbindemittel besteht oder diese Bestandteile aufweist,
oder
- Verwenden einer lösungsmittellöslichen, bindemittelhaltigen Folie als elektrisches Leitelement (3), Anlösen dieser Folie durch ein geeignetes Lösungsmittel und Auflegen des elektrischen Leiters (6) des Anschlusskabels (4) auf die angelösten Bereiche, sodass der elektrische Leiter (6) durch das Trocknen des Bindemittels der Folie mit dem elektrischen Leitelement (3) verbunden wird.

2. Verfahren nach Anspruch 1, **dadurch gekennzeichnet, dass** als elektrisches Leitelement (3) eine lösungsmittellösliche, bindungsmittelhaltige Karbonfolie verwendet wird.

## Claims

1. A method for producing a bioelectrode comprising a skin-side, electrically conducting adhesive layer (7) and a flexible electrical connecting cable (4) which in an electrically insulating cable sheath (5) includes at least one electrical conductor (6) in the form of a braid having a plurality of individual wires conducting individual fibers, **characterized by** the steps:
- fan-open the individual wires or individual fibers of the braid,
- preassembling an electrical conducting element (2, 3) at the electrode-side end (4a) of the connecting cable (4), whereby the electrical conducting element (2, 3) is connected to the fanned-open individual wires or individual fibers of the braid of the electrical conductor (6) of the connecting cable (4), wherein the electrical conducting element (2, 3) and the electrical conductor (6) arranged therein in the bioelectrode are of a layer configuration, with a joint layer thickness of between 50 and 250 micrometers, and
- subsequently electrically connecting the preassembled electrical conducting element (2, 3) - if applicable by an interconnection of at least one further electrical conducting element (3, 8) - to the skin-side, electrically conducting adhesive layer (7),
wherein it is additionally carried out
- that the preassembled electrical conducting element (2, 3) is formed of an electrical conductive layer, that the layer of the preassembled electrical conducting element (2, 3) comprises an electrically conductive thermoplastic material, and that the electrical conductor (6) of the connecting cable (4) is welded to the thermoplastic, electrically conductive layer, wherein the welded layer forms the preassembled electrical conducting element (2),
or
- providing the electrical conducting element (3) or the electrical conductor (6) of the connecting layer (4) with an electrical conductive lacquer layer, and placing the electrical conductor (6) of the connecting cable (4) on the electrical conducting element (3) so that the electrical conductor (6) is connected to the electrical conducting element (3) by drying of the lacquer layer, wherein the lacquer of the lacquer layer consists of polyurethane binding agent or polyvinyl chloride binding agent or can include such constituents,
or
- using a solvent-soluble, binding agent-bearing film is as the electrical conducting element (3), solubilising this film by a suitable solvent, and placing the electrical conductor (6) of the connecting cable (4) on the solubilised regions so that the electrical conductor (6) is connected to the electrical conducting element (3) by drying of the binding agent of the film.

2. The method according to claim 1, **characterized in that** a solvent-soluble, binding agent-bearing carbon film is used as the electrical conducting element (3).

## Revendications

1. Procédé de fabrication d'une bioélectrode, avec une couche adhésive (7) électriquement conductrice, côté peau et avec un câble de raccordement électrique flexible (4) qui comprend, dans une gaine de câble isolée électriquement (5), au moins un conducteur électrique (6) sous forme de toron composé de plusieurs brins ou monofilaments conducteurs, **caractérisé par** les étapes de :
- répartition des brins ou monofilaments du toron,
- prémontage d'un élément conducteur électrique (2, 3) à l'extrémité côté électrode (4a) du câble de raccordement (4), moyennant quoi l'élément conducteur (2, 3) est raccordé aux brins ou monofilaments répartis du toron du conducteur électrique (6) du câble de raccordement (4), dans lequel l'élément conducteur électrique (2, 3) et le conducteur électrique (6) qui y est agencé dedans sont réalisés dans la bioélectrode sous forme de couche avec une épaisseur de couche commune entre 50 et 250 micromètres, et
- ensuite raccordement électrique de l'élément conducteur électrique (2, 3) prémonté, le cas échéant par interconnexion d'au moins une autre couche électriquement conductrice (3, 8), à la couche adhésive (7) électriquement conductrice, côté peau,
dans lequel il est réalisé en outre :
- que l'élément conducteur électrique (2, 3) prémonté est formé d'une couche électriquement conductrice, que la couche de l'élément conducteur électrique (2) prémonté se compose d'un matériau thermoplastique électriquement conducteur et que le conducteur électrique (6) du câble de raccordement (4) est soudé avec la couche thermoplastique électriquement conductrice, dans lequel la couche soudée forme l'élément conducteur électrique (2) prémonté,
ou
- équipement de l'élément conducteur électrique (3) ou du conducteur électrique (6) du câble de raccordement (4) d'une couche de vernis électriquement conductrice et pose du conducteur électrique (6) du câble de raccordement (4) sur l'élément conducteur électrique (3) de sorte que le conducteur électrique (6) soit raccordé par le séchage de la couche de vernis à l'élément conducteur électrique (3),
dans lequel le vernis de la couche de vernis se compose de liant de polyuréthane ou liant de polychlorure de vinyle ou présente ces composants,
ou
- utilisation d'une feuille contenant un liant, soluble par solvant en tant qu'élément conducteur électrique (3), dissolution de cette feuille par un solvant adapté et pose du conducteur électrique (6) du câble de raccordement (4) sur les zones dissoutes de sorte que le conducteur électrique (6) soit raccordé par le séchage du liant de la feuille à l'élément conducteur électrique (3).

2. Procédé selon la revendication 1, **caractérisé en ce qu'**une feuille de carbone contenant un liant, soluble par solvant est utilisée en tant qu'élément conducteur électrique (3).
